(19) Europäisches Patentamt

European Patent Office

Office européen des brevets

(11) **EP 0 987 016 B1**

(12) **FASCICULE DE BREVET EUROPEEN**

(45) Date de publication et mention
de la délivrance du brevet:
**10.04.2002 Bulletin 2002/15**

(51) Int Cl.⁷: **A61K 7/48**

(21) Numéro de dépôt: **99402160.8**

(22) Date de dépôt: **31.08.1999**

(54) **Composition anti-rides comprenant une association de polymères tenseurs d'origine synthétique et/ou naturelle et de polymères dendritiques**

Antifaltenmittel enthaltend eine Mischung von Tensorpolymeren synthetischen und/oder natürlichen Ursprungs und dendritischen Polymeren

Antiwrinkle composition containing a combination of tensor polymers from synthetic and/or natural origin and dendritic polymers

(84) Etats contractants désignés:
**AT BE CH CY DE DK ES FI FR GB GR IE IT LI LU MC NL PT SE**

(30) Priorité: **17.09.1998 FR 9811635**

(43) Date de publication de la demande:
**22.03.2000 Bulletin 2000/12**

(73) Titulaire: **L'OREAL**
**75008 Paris (FR)**

(72) Inventeurs:
• **Simon, Pascal**
**94400 Vitry/s/Seine (FR)**
• **Chevalier, Véronique**
**94440 Villecresnes (FR)**

(74) Mandataire: **Rasson, Catherine**
**L'OREAL-DPI**
**6 rue Bertrand Sincholle**
**92585 Clichy Cedex (FR)**

(56) Documents cités:
**WO-A-93/17060        FR-A- 2 758 083**

**Description**

La présente invention concerne des compositions cosmétiques ou dermatologiques anti-rides comprenant des polymères à effet "tenseur" associés à des polyesters dendritiques, un procédé de traitement cosmétique utilisant ces compositions ainsi que l'utilisation de l'association d'un système polymérique filmogène à effet "tenseur" et d'un polyester dendritique pour fabriquer des compositions cosmétiques ou dermatologiques destinées à diminuer et/ou effacer les rides et/ou les ridules de la peau par un effet tenseur.

Le processus de vieillissement de la peau s'accompagne d'une modification progressive de la structure et des fonctions cutanées. Les principaux signes cliniques du vieillissement cutané sont l'apparition de rides et de ridules qui augmentent avec l'âge.

Il est connu de remédier à ces signes du vieillissement en utilisant des compositions cosmétiques ou dermatologiques contenant des principes actifs tels que les $\alpha$-hydroxy-acides, les $\beta$-hydroxy-acides et les rétinoïdes. Ces molécules agissent sur les rides en éliminant les cellules mortes et en accélérant le processus de renouvellement cellulaire. Toutefois, l'effet visible de ces compositions ne se produit qu'au bout d'un certain temps d'application, pouvant aller de quelques jours à plusieurs semaines.

Une approche pour résoudre ce problème a consisté à utiliser des agents dits "tenseurs" qui, par un effet de tension de la couche superficielle de la peau, sont capables de lisser la peau en diminuant le nombre et la profondeur des rides et ridules et de faire disparaître les marques de fatigue, et ceci de façon instantanée.

Ces "agents tenseurs" sont des polymères d'origine naturelle ou synthétique capables de former un film provoquant la rétraction du *stratum corneum,* la couche cornée superficielle de l'épiderme.

L'utilisation cosmétique ou dermatologique de tels systèmes polymériques pour atténuer les effets du vieillissement de la peau est décrite dans les demandes de brevets FR-2 758 083 et FR-2 758 084.

Ces systèmes polymériques tenseurs, bien que très efficaces et rapides, provoquent cependant parfois une impression d'inconfort chez certains utilisateurs, notamment ceux ayant la peau fragile. Ces agents tenseurs forment en effet sur la peau un film trop rigide et peu souple, Le problème s'est donc posé de chercher à obtenir un effet tenseur important en utilisant des quantités plus faibles de polymères tenseurs.

La demanderesse a découvert de manière surprenante qu'en associant des polyesters dendritiques à groupements terminaux hydroxyle qui, par eux-mêmes, n'ont aucun pouvoir tenseur de l'épiderme, aux systèmes polymériques tenseurs connus, il était possible de renforcer de manière significative l'effet tenseur de ces derniers.

La présente invention a donc pour objet des compositions cosmétiques ou dermatologiques anti-rides renfermant un système polymérique filmogène contenant au moins un polymère à effet "tenseur", d'origine naturelle et/ou synthétique, et un polyester dendritique à groupements hydroxyle terminaux.

L'invention a également pour objet un procédé de traitement non thérapeutique de la peau avec les compositions cosmétiques anti-rides ci-dessus, ainsi que l'utilisation de l'association d'un système polymérique filmogène à effet "tenseur" et d'un polyester dendritique pour fabriquer des compositions cosmétiques ou dermatologiques destinées à diminuer et/ou effacer les rides et/ou les ridules de la peau.

D'autres objets apparaîtront à la lecture de la description et des exemples qui suivront.

L'effet cosmétique des compositions de la présente invention repose sur leur pouvoir "tenseur" de la peau.

Ce pouvoir "tenseur" est défini selon la présente invention comme la capacité d'un film homogène formé sur le *stratum corneum* isolé, à provoquer une rétraction d'au moins 1 % et de préférence d'au moins 1,3 % de celui-ci à une température de 30 °C et pour une humidité relative de 40 %. Selon la présente invention, ce film est formé par application et séchage d'une dispersion aqueuse contenant 7 % dudit système polymérique filmogène. Pour déterminer l'effet tenseur, on mesure la longueur de l'échantillon de *stratum corneum* avant traitement et celle obtenue après traitement, c'est-à-dire après application du polymère. L'effet tenseur est caractérisé par la diminution de la longueur de l'échantillon après traitement, rapportée à la longueur initiale de l'échantillon avant traitement, selon l'équation :

$$\text{effet tenseur} = 100 \times \frac{l_1 - l_0}{l_0}\%$$

$l_1$ étant la longueur après traitement et $l_0$ la longueur avant traitement.

La longueur de l'échantillon est mesurée avec l'appareil "Miniature Tensile tester MTT 610".

On appelle polymère "filmogène" selon la présente invention, tout polymère ou système polymérique capable de former, après application sur une plaque de verre et séchage, un film continu ne formant pas d'écailles ni de craquelures.

Le film formé par le système polymérique tenseur contenu dans les compositions anti-rides de la présente invention doit avoir un module d'élasticité (module d'Young déterminé par des méthodes de micro- ou nano-indentation instrumentée ; norme ASTM E384-89) allant de $10^8$ à $10^{10}$ N/m$^2$ et de préférence de 6,5 à $9.10^9$ N/m$^2$1. Les valeurs du module d'élasticité comprises dans cet intervalle sont de 10 à 100 fois supérieures à celle du *stratum corneum.* Un tel module d'élasticité permet donc au film polymère de suivre parfaitement les déformations de la peau ce qui aboutit à la fois à un efficacité persistante et un bon confort d'utilisation dû à l'absence d'une impression de tiraillement excessif.

Le film formé sur l'épiderme doit par ailleurs être perméable à la vapeur d'eau pour ne pas entraver la transpiration cutanée. La perméabilité du film est évaluée par mesure de la *Perte Insensible en Eau* (PIE) du *stratum corneum* dégraissé et traité par le polymère. Lorsque le film est suffisamment perméable à la vapeur d'eau la PIE n'est pas modifiée. La mesure de la PIE s'effectue de manière classique à l'aide d'un évaporimètre (Servomed) qui permet une détermination quantitative de l'évaporation d'eau, c'est-à-dire du transport d'eau par diffusion sur un échantillon de *stratum corneum* obturant une capsule cylindrique contenant de l'eau, le tout étant placé dans une chambre à température et humidité relative contrôlées. Des capteurs permettent de mesurer la pression partielle de vapeur d'eau en deux points situés à des distances différentes de l'échantillon. On détermine ainsi le gradient de pression partielle de vapeur d'eau entre les deux points, et donc le taux d'évaporation conformément à la loi de Fick.

Les polymères tenseurs filmogènes remplissant les critères d'élasticité, de rétraction et de perméabilité à la vapeur d'eau décrits ci-dessus sont des polymères d'origine naturelle et/ou d'origine synthétique.

Parmi les polymères naturels à effet tenseur, on peut citer les polymères d'origine végétale, les polymères issus des phanères, les protéines de l'oeuf, les latex d'origine naturelle et les polysaccharides.

Les polymères d'origine végétale sont par exemple les extraits protéiques de céréales, de légumineuses et d'oléagineuses tels que les extraits de maïs, de seigle, de froment, de sarrasin, de sésame, d'épeautre, de pois, de fève, de lentille, de soja et le lupin. Comme protéines appropriées, on peut citer par exemple l'extrait protéique de soja vendu par la société ISD sous la dénomination PROFAM 972 ou par la société LSN sous la dénomination ELESERYL, ou la fraction protéique du lupin blanc.

Comme polymères dérivés des phanères, on peut utiliser tout polymère provenant des poils, ongles, carapaces d'insectes ou de crustacés, cheveux, plumes, becs, sabots et crêtes d'animaux. On peut citer par exemple la chitine et ses dérivés, notamment le chitosane, ainsi que les dérivés du chitosane tels que l'hydroxypropylchitosane, le dérivé succinylé du chitosane, le lactate de chitosane, le glutamate de chitosane ou le succinamide de carboxyméthylchitosane, ou encore les dérivés de la kératine tels que les hydrolysats de kératine et les kératines sulfoniques.

Comme protéine de l'oeuf on peut citer l'albumine de l'oeuf.

Les latex naturels sont par exemple la résine shellac, la gomme de sandaraque, les dammarts, les élémis, les copals, les dérivés cellulosiques et les mélanges de ces polymères.

Les polysaccharides sont par exemple la gomme de guar, éventuellement modifiée par des groupements anioniques tels que carboxylate ou phosphate, la gomme de xanthane, les alginates.

Les polymères tenseurs filmogènes d'origine synthétique se présentent sous forme d'un latex ou d'un pseudolatex.

Un latex est une suspension aqueuse de particules de polymères obtenus par polymérisation radicalaire ou par polycondensation selon la technique bien connue de polymérisation en émulsion.

On entend par pseudolatex une dispersion aqueuse de polymères synthétiques obtenue non pas par polymérisation en émulsion mais par dispersion dans de l'eau de polymères obtenus préalablement selon une autre technique de synthèse.

Dans les deux cas les dispersions sont généralement stabilisées par des charges portées par le polymère.

Les particules de latex ou de pseudolatex ont de préférence une dimension allant de 10 à 400 nm, de préférence de 20 à 350 nm.

Les polymères synthétiques formant les particules de latex ou de pseudolatex sont par exemple les polyuréthannes anioniques, cationiques, non ioniques ou amphotères, les polyuréthanne-polyacryliques, polyuréthanne-polyvinylpyrrolidones, polyuréthanne-polyesters, polyuréthanne-polyéthers, polyurées.

Le polyuréthanne peut être par exemple un copolymère polyuréthanne, polyurée/uréthanne ou polyurée, aliphatique, cycloaliphatique ou aromatique, comportant

- au moins une séquence polyester aliphatique linéaire ou ramifiée et/ou cycloaliphatique et/ou aromatique et/ou,
- au moins une séquence polyéther aliphatique et/ou cycloaliphatique et/ou aromatique et/ou
- au moins une séquence siliconée, substituée ou non, linéaire ou ramifiée, et/ou
- au moins une séquence comportant des groupes fluorés.

Les polyuréthannes peuvent également être obtenus à partir de polyesters linéaires ou ramifiés, ou à partir de résines alkyd comportant des atomes d'hydrogène mobiles que l'on modifie par réaction avec un diisocyanate et un composé organique bifonctionnel (par exemple dihydrogéno, diamino ou hydroxyaminé) comportant en plus soit un groupement acide carboxylique ou carboxylate, soit un groupement acide sulfonique ou sulfonate, soit encore un groupement amine tertiaire ou un groupement ammonium quaternaire.

On peut également citer comme polymères synthétiques formant les latex ou pseudolatex, les polyesters, les poly(ester amide), les polyesters à chaîne grasse, les polyamides et les résines époxyesters.

On peut citer comme comonomères anioniques permettant de synthétiser des polyuréthannes chargés négativement par exemple l'acide diméthylolpropionique, l'acide ou l'anhydride triméllitique, le pentanediol-3-sulfonate de sodium ou le 1,3-dicarboxybenzène-5-sulfonate de sodium.

Ces polymères sont en particulier ceux décrits dans le document EP-A-648 485.

Les latex ou pseudolatex peuvent également être constitués d'homopolymères ou copolymères acryliques, ou encore de polymères à base d'acide isophtalique sulfoné.

Ces polymères tenseurs filmogènes d'origine synthétique sont commercialisés par exemple sous les dénominations SANCURE 2060 (polyester-polyuréthanne), SANCURE 2255 (polyester-polyuréthanne), SANCURE 815 (polyester-polyuréthanne), SANCURE 878 (polyéther-polyuréthanne) et SANCURE 861 (polyéther-polyuréthanne) par la société SANNCOR, sous les dénominations NEOREZ R974 (polyester-polyuréthanne), NEOREZ R981 (polyester-polyuréthanne), NEOREZ R970 (polyéther-polyuréthanne) par la société ICI, et sous la dénomination NEOCRYL XK-90 (dispersion d'un copolymère acrylique) par la société ZENECA.

Parmi l'ensemble de polymères tenseurs d'origine naturelle et synthétique présentés ci-dessus, on utilisera dans la présente invention de préférence des polymères comportant des groupements hydroxyle libres ou des groupements anioniques.

Le système polymérique filmogène peut être formé par un mélange de plusieurs des polymères tenseurs d'origine naturelle et/ou d'origine synthétiques décrits ci-dessus. Il peut en outre contenir un ou plusieurs agents plastifiants choisi(s) de façon à obtenir les caractéristiques mécaniques recherchées.

L'agent plastifiant peut être choisi parmi tous les composés connus de l'homme du métier comme étant susceptibles de remplir cette fonction. On peut citer par exemple, les alcools inférieurs (éthanol, propanol, butanol) les glycols et dérivés des glycols tels que l'éther éthylique ou méthylique de diéthylèneglycol, l'éther éthylique ou butylique d'éthylèneglycol, l'éther méthylique ou phénylique de propylèneglycol, l'éther éthylique ou butylique de dipropylèneglycol, l'éther butylique ou méthylique de tripropylèneglycol; les esters de glycérol, les esters d'acides tels que les citrates, phtalates, adipates, carbonates, tartrates, phosphates ou sébaçates ; les dérivés oxyéthylénés tels que les huiles oxyéthylénées, par exemple l'huile de ricin oxyéthylénée, et les huiles de silicone oxyéthylénées ; les polymères hydrosolubles ou en dispersion aqueuse ayant une température de transition vitreuse inférieure à 25 °C, de préférence inférieure à 15 °C .

La quantité d'agent plastifiant est choisie par l'homme de métier sur la base de ses connaissances générales, de manière à obtenir un système polymérique conduisant à un film ayant les propriétés mécaniques souhaitées, tout en conservant à la composition des propriétés cosmétiques acceptables.

Dans les compositions anti-rides de la présente invention, ce système polymère filmogène à effet tenseur, comprenant un ou plusieurs polymères d'origine naturelle et/ou synthétique et éventuellement un agent plastifiant, et se présentant sous forme d'une dispersion aqueuse, est associé à des polyesters d'un type particulier, à savoir des polyesters ayant une structure très ramifiée appelée "dendritique".

Les polymères dendritiques ou dendrimères (du grec *dendron* = arbre) sont des molécules polymères "arborescentes", c'est-à-dire très ramifiées inventées par D. A. Tomalia et son équipe au début des années 90 (Donald A. Tomalia *et al.*, *Angewandte Chemie, Int. Engl. Ed.,* vol. 29, n° 2, pages 138 - 175). Il s'agit de structures moléculaires construites autour d'un motif central généralement polyvalent. Autour de ce motif central sont enchaînés, en couches concentriques et selon une structure parfaitement déterminée, des motifs ramifiés d'allongement de chaîne donnant ainsi naissance à des macromolécules symétriques, monodispersées ayant une structure chimique et stéréochimique bien définie.

Les polymères dendritiques utilisés dans les compositions cosmétiques ou dermatologiques de la présente invention sont des dendrimères ayant la structure chimique d'un polyester et qui sont terminés par des groupements hydroxyle. La structure et la préparation de ces polymères est décrite dans la demande de brevet WO-A-93/17060.

Plus précisément, les polymères dendritiques utilisés dans les compositions de la présente invention peuvent être définis comme étant des macromolécules hautement ramifiées de type polyester, constituées

- d'un motif central dérivé d'un composé initiateur portant un ou plusieurs fonctions hydroxyle (a),
- de motifs d'allongement de chaîne dérivés d'une molécule d'allongement de chaîne portant une fonction carboxyle (b) et au moins deux fonctions hydroxyle (c),

chacune des fonctions hydroxyle (a) de la molécule centrale étant le point de départ d'une réaction de polycondensation (par estérification) qui débute par la réaction des fonctions hydroxyle (a) de la molécule centrale avec les fonctions carboxyle (b) des molécules d'allongement de la chaîne, puis se poursuit par réaction des fonctions carboxyle (b) avec les fonctions hydroxyle (c) des molécules d'allongement de la chaîne.

Le composé initiateur portant une ou plusieurs fonctions hydroxyle et formant le motif central autour duquel se construira la structure dendritique est un composé mono-, di- ou polyhydroxylé. Il est généralement choisi parmi

(a) un alcool monofonctionnel,
(b) un diol aliphatique, cycloaliphatique ou aromatique,
(c) un triol,
(d) un tétrol,
(e) un alcool de sucre,
(f) l'anhydro-ennéa-heptitol ou le dipentaérythritol,
(g) un $\alpha$-alkylglycoside,
(h) un polymère polyalcoxylé obtenu par polyalcoxylation d'un des alcools (a) à (g), ayant une masse molaire au plus égale à 8000.

On peut citer à titre d'exemples de composés initiateurs préférés servant à préparer les polyesters dendritiques utilisés dans la présente invention le ditriméthylolpropane, le ditriméthyloléthane, le dipentaérythritol, le pentaérythritol, un pentaérythritol alcoxylé, le triméthyloléthane, le triméthylolpropane, un triméthylolpropane alcoxylé, le glycérol, le néopentylglycol, le diméthylolpropane ou le 1,3-dioxane-5,5-diméthanol.

On fait réagir sur ces composés inititateurs hydroxylés formant le motif central du futur dendrimère, des molécules dites molécules d'allongement de chaîne qui sont des composés de type diol-monoacide choisis parmi

- les acides monocarboxyliques comportant au moins deux fonctions hydroxyle, et
- les acides monocarboxyliques comportant au moins deux fonctions hydroxyle dont une ou plusieurs porte(nt) un substituant hydroxyalkyle.

Des exemples préférés de tels composés sont l'acide diméthylolpropionique, l'acide $\alpha,\alpha$-bis(hydroxyméthyl)-butyrique, l'acide $\alpha,\alpha,\alpha$-tris(hydroxyméthyl)-acétique, l'acide $\alpha,\alpha$-bis(hydroxyméthyl)-valérique, l'acide $\alpha,\alpha$-bis(hydroxy)-propionique et l'acide 3,5-dihydroxybenzoïque.

Dans un mode de réalisation particulièrement préféré de la présente invention, le composé initiateur est choisi parmi le ditriméthylolpropane, le triméthylolpropane, un pentaérythritol éthoxylé, le pentaérythritol ou le glycérol, et la molécule d'allongement de chaîne est l'acide diméthylolpropionique.

Les polymères dendritiques de type polyester à fonctions hydroxyle terminales utilisés dans les compositions de la présente invention sont caractérisés en outre par le fait qu'une partie des fonctions hydroxyle terminales du polymère dendritique de type polyester peuvent porter des substituants dérivés d'au moins un agent de terminaison de chaîne.

On utilise de préférence un polymère sans substituants dérivés. Toutefois, lorqu'une partie des fonctions hydroxyle terminales portent un substituant dérivé, la fraction de ces fonctions hydroxyle terminales portant un motif de terminaison de chaîne est généralement comprise entre 1 et 90 % en moles, de préférence entre 10 et 50 % en moles rapporté au nombre total de fonctions hydroxyle terminales.

Le choix approprié d'un agent de terminaison de chaîne approprié permet de modifier à souhait les propriétés physico-chimiques des polyesters dendritiques utilisés dans les compositions de la présente invention.

Ledit agent de terminaison de chaîne peut être choisi dans une grande variété de composés capables de former des liaisons covalentes avec les fonctions hydroxyle terminales. Ces composés englobent notamment

i) un acide monocarboxylique saturé ou un acide gras saturé ou un anhydride d'un tel composé,
ii) un acide gras insaturé,
iii) un acide monocarboxylique insaturé,
iv) un diisocyanate ou un oligomère d'un tel composé,
v) un produit d'addition préparé à partir d'un composé selon iv),
vi) un acide dicarboxylique ou polycarboxylique ou un anhydride d'un tel composé,
vii) un produit d'addition préparé à partir d'un composé selon vi),
viii) un acide monocarboxylique aromatique,
ix) une épihalogénhydrine,
x) un ester glycidylique d'un acide monocarboxylique ou d'un acide gras comportant de 1 à 24 atomes de carbone,
xi) un époxyde d'un acide gras insaturé comportant de 3 à 24 atomes de carbone.

Des composés de terminaison de chaîne préférés sont en particulier l'acide laurique, les acides gras de graines de lin, les acides gras de soja, les acides gras de suif, les acides gras d'huile de ricin déshydrogéné, l'acide caproïque, l'acide caprylique, le maléate de l'éther diallylique de triméthylolpropane, l'acide méthacrylique et l'acide acrylique.

Les polymères dendritiques de type polyester à fonctions hydroxyle terminales et portant éventuellement des groupements de terminaison de chaîne sont connus et sont commercialisés par la société PERSTORP.

Des polymères particulièrement préférés utilisés dans la présente invention sont

- un polyester dendritique obtenu par polycondensation d'acide diméthylolpropionique sur du triméthylolpropane, exempt d'agents de terminaison de chaîne commercialisé sous la dénomination BOLTORN H40 TMP CORE;
- un polyester dendritique obtenu par polycondensation d'acide diméthylolpropionique sur du pentaérythritol polyoxyéthyléné (5 motifs d'OE sur chaque fonction hydroxyle), dont 50 % des fonctions hydroxyle sont estérifiées par de l'acide caprique/caprylique (nom technique HBP 3G estérifié) ;
- un polyester dendritique obtenu par polycondensation d'acide diméthylolpropionique sur du pentaérythritol polyoxyéthyléné (5 motifs d'OE sur chaque fonction hydroxyle), exempt d'agent de terminaison de chaîne (nom technique HBP Polyol 3G),

tous ces polymères étant des produits de la société PERSTORP.

La concentration du polymère dendritique rapportée à la composition totale est comprise dans l'intervalle allant de 0,1 à 5 % en poids.

Le système polymérique filmogène est présent à raison de 0,5 à 70 % en poids, de préférence de 0,5 à 30 % en poids, exprimé en matière active rapportée à la composition totale.

On choisira les quantités de polymère dendritique et de polymères tenseurs de manière à ce que le rapport en poids du polymère dendritique au système polymérique filmogène soit compris dans l'intervalle allant de 1/2 à 1/100, de préférence de 1/10 à 1/50.

Les compositions anti-rides selon l'invention peuvent contenir en outre un ou plusieurs principes actifs cosmétiques choisis parmi les agents anti-radicaux libres, les agents hydratants, les vitamines, les protéines autres que celles constituant le système tenseur, les céramides, les $\alpha$-hydroxy-acides, les $\beta$-hydroxyacides et les rétinoïdes.

Elles peuvent contenir en outre des adjuvants cosmétiques usuels en cosmétique et dermatologie. Ces adjuvants sont par exemple les solvants, les agents régulateurs de pH, les anti-oxydants, les agents conservateurs, les pigments et colorants, les charges, les émollients, les agents anti-mousse, les corps gras tels que huiles ou cires végétales ou animales, les silicones, les parfums, les agents tensioactifs, les plastifiants, les polymères épaississants ou gélifiants, les filtres solaires et les agents apportant de la douceur (allantoïne, PVM/MA décadiène copolymer).

Comme huiles utilisables dans la composition de l'invention, on peut notamment citer par exemple les huiles d'origine végétale, les huiles minérales (huile de vaseline), les huiles de synthèse, les huiles de silicone (cyclométhicone) et les huiles fluorées. Les autres corps gras susceptibles d'être présents dans la phase huileuse peuvent être par exemple les acides gras, les alcools gras (alcool cétylique) et les cires.

Bien entendu, l'homme de métier veillera à choisir ce ou ces éventuels composés complémentaires et leur quantité, de manière telle que les propriétés avantageuses attachées intrinsèquement à la composition cosmétique ou dermatologique conforme à l'invention ne soient pas, ou substantiellement pas, altérées par la ou les adjonctions envisagées.

Les compositions anti-rides peuvent se présenter sous n'importe quelle forme permettant la formation d'un film homogène ayant l'effet cosmétique tenseur souhaité. Il s'agit par exemple d'une émulsion telle qu'une crème ou un lait, d'un gel, d'une lotion, d'une dispersion vésiculaire, d'un sérum, d'une pâte ou d'un bâtonnet solide.

La présente invention concerne également un procédé de traitement non thérapeutique de la peau qui consiste à appliquer les compositions décrites ci-dessus en une mince couche sur la peau du visage, du cou et/ou du décolleté.

Elle concerne également l'utilisation d'une association d'une dispersion d'un système polymérique filmogène contenant au moins un polymère d'origine naturelle et/ou synthétique et capable de former un film perméable à la vapeur d'eau, présentant un module d'Young allant de $10^8$ à $10^{10}$ N/m² et produisant, après application à une concentration de 7 % dans l'eau puis séchage, une rétraction du *stratum corneum* isolé supérieure à 1 % à une température de 30 °C et pour une humidité relative de 40 %, et d'un polymère dendritique de type polyester à fonctions hydroxyle terminales, pour préparer une composition cosmétique ou dermatologique destinée à diminuer et/ou effacer les rides et/ou ridules de la peau.

L'invention est illustrée plus en détail dans les exemples suivants.

## Exemple 1

| Crème anti-rides | |
|---|---|
| alcool cétylique | 1,5 % |
| huile de vaseline | 5 % |
| cyclométhicone | 7 % |
| tristéarate de sorbitane (tensio-actif) | 1,3 % |
| stéarate de PEG 40 (tensio-actif) | 2,7 % |
| protéine de soja (Eleseryl® commercialisé par LSN) | 3 % |
| Sancure 2060 waterborne urethane (27 % de matière active dans l'eau) | 10 % |
| BOLTORN H 40 TMP commercialisé par PERSTORP | 0,2 % |
| alcool éthylique | 10 % |
| parfums, conservateurs | q.s. |
| eau déminéralisée | qsp 100 % |

## Exemple 2

| Crème anti-rides | |
|---|---|
| alcool cétylique | 1,5 % |
| huile de vaseline | 5 % |
| cyclométhicone | 7 % |
| tristéarate de sorbitane (tensio-actif) | 1,3 % |
| stéarate de PEG 40 (tensio-actif) | 2,7 % |
| fraction protéique de lupin blanc (à 0,7 % de matière active) | 30 % |
| Sancure 815 (35 % de matière active dans l'eau) | 12 % |
| BOLTORN H 40 TMP commercialisé par PERSTORP | 0,2 % |
| alcool éthylique | 10 % |
| parfums, conservateurs | q. s. |
| eau déminéralisée | qsp 100 % |

## Exemple 3

| Sérum | |
|---|---|
| polyacrylamide/$C_{13}$-$C_{14}$ Isoparaffine/Laureth-7 (Sepigel® commercialisé par SEPPIC) | 1 % |
| gomme xanthane | 0,2 % |
| PVM/MA decadiene cross polymer (ANTARON ST 06 commercialisé par ISP) | 0,2 % |
| triéthanolamine | 0,2 % |
| SANCURE 815 | |

## EP 0 987 016 B1

(suite)

| Sérum | |
|---|---|
| (35 % de matière active dans l'eau) | 12 % |
| BOLTORNH 40 TMP | 0,3 % |
| commercialisé par PERSTORP | |
| alcool éthylique | 10 % |
| parfums, conservateurs | q. s. |
| eau déminéralisée | qsp 100 % |

**Revendications**

1. Composition cosmétique ou dermatologique anti-rides, **caractérisée par le fait qu'**elle comprend, dans un milieu physiologiquement acceptable, l'association

   - d'une dispersion d'un système polymérique filmogène contenant au moins un polymère d'origine naturelle et/ou synthétique et capable de former un film perméable à la vapeur d'eau, présentant un module d'Young allant de $10^8$ à $10^{10}$ N/m$^2$ et produisant, après application à une concentration de 7 % dans l'eau puis séchage, une rétraction du *stratum corneum* isolé supérieure à 1 % à une température de 30 °C et pour une humidité relative de 40 %, et
   - d'un polymère dendritique de type polyester à fonctions hydroxyle terminales.

2. Composition anti-rides selon la revendication 1, **caractérisée par le fait que** le polymère contenu dans ledit système polymérique filmogène est un polymère d'origine naturelle.

3. Composition anti-rides selon la revendication 2, **caractérisée par le fait que** le polymère d'origine naturelle est choisi parmi les polymères d'origine végétale, les polymères issus des phanères, les protéines de l'oeuf et les latex d'origine naturelle.

4. Composition anti-rides selon la revendication 3, **caractérisée par le fait que** le polymère d'origine naturelle de type latex est choisi parmi la résine shellac, la gomme de sandaraque, les dammarts, les élémis, les copals, les dérivés cellulosiques et les mélanges de ces polymères.

5. Composition anti-rides selon la revendication 3, **caractérisée par le fait que** le polymère issu des phanères est choisi parmi la chitine, le chitosane, l'hydroxypropylchitosane, le dérivé succinylé du chitosane, le lactate de chitosane, le glutamate de chitosane, le succinamide de carboxyméthylchitosane, hydrolysats de kératine et les kératines sulfoniques.

6. Composition anti-rides selon la revendication 3, **caractérisée par le fait que** le polymère d'origine végétale est choisi parmi les extraits protéiques de céréales, de légumineuses et d'oléagineuses.

7. Composition anti-rides selon la revendication 6, **caractérisée par le fait que** l'extrait est choisi parmi les extraits protéiques de maïs, de seigle, de froment, de sarrasin, de sésame, d'épeautre, de pois, de fève, de lentille, de soja et de lupin.

8. Composition anti-rides selon la revendication 1, **caractérisée par le fait que** le polymère contenu dans ledit système polymérique filmogène est un polymère synthétique se présentant sous forme d'un latex ou pseudolatex.

9. Composition anti-rides selon la revendication 8, **caractérisé par le fait que** le polymère synthétique est choisi parmi les polyuréthannes anioniques, cationiques, non ioniques ou amphotères, les polyuréthanne-polyacrylique, polyuréthanne-polyvinylpyrrolidone, polyuréthanne-polyester, polyuréthanne-polyéther, polyurées, les homo- ou copolymères acryliques, les polymères d'acide isophtalique sulfoné et leurs mélanges.

10. Composition anti-rides selon l'une quelconque des revendications précédentes, **caractérisée par le fait que** le polymère d'origine synthétique et/ou naturelle contenu dans ledit système polymérique filmogène comporte des groupements hydroxyle libres ou des groupements anioniques.

8

**11.** Composition anti-rides selon l'une quelconque des revendications précédentes, **caractérisée en ce que** le système polymérique comprend en outre un agent plastifiant.

**12.** Composition anti-rides selon l'une quelconque des revendications précédentes, **caractérisée par le fait que** le polymère dendritique à fonctions hydroxyle terminales est une macromolécule hautement ramifiée de type polyester, constituée

- d'un motif central dérivé d'un composé initiateur portant une ou plusieurs fonctions hydroxyle (a),
- de motifs d'allongement de chaîne dérivés d'une molécule d'allongement de chaîne portant une fonction carboxyle (b) et au moins deux fonctions hydroxyle (c),

chacune des fonctions hydroxyle (a) de la molécule centrale étant le point de départ d'une réaction de polycondensation (polyestérification) qui débute par la réaction des fonctions hydroxyle (a) de la molécule centrale avec les fonctions carboxyle (b) des molécules d'allongement de la chaîne, puis se poursuit par réaction des fonctions carboxyle (b) avec les fonctions hydroxyle (c) des molécules d'allongement de la chaîne.

**13.** Composition cosmétique anti-rides selon la revendication 12, **caractérisée par le fait que** le composé initiateur portant une ou plusieurs fonctions hydroxyle formant le motif central est choisi parmi

(a) un alcool monofonctionnel,
(b) un diol aliphatique, cycloaliphatique ou aromatique,
(c) un triol,
(d) un tétrol,
(e) un alcool de sucre,
(f) l'anhydro-ennéa-heptitol ou le dipentaérythritol,
(g) un $\alpha$-alkylglycoside,
(h) un polymère polyalcoxylé obtenu par polyalcoxylation d'un des alcools (a) à (g), ayant une masse molaire au plus égale à 8000.

**14.** Composition cosmétique anti-rides selon la revendication 13, **caractérisée par le fait que** le composé initiateur est choisi parmi le ditriméthylolpropane, le ditriméthyloléthane, le dipentaérythritol, le pentaérythritol, un pentaérythritol alcoxylé, le triméthyloléthane, le triméthylolpropane, un triméthylolpropane alcoxylé, le glycérol, le néopentylglycol, le diméthylolpropane ou le 1,3-dioxane-5,5-diméthanol.

**15.** Composition cosmétique anti-rides selon l'une quelconque des revendications 12 à 14, **caractérisée par le fait que** la molécule d'allongement de chaîne est choisie parmi

- les acides monocarboxyliques comportant au moins deux fonctions hydroxyle,
- les acides monocarboxyliques comportant au moins deux fonctions hydroxyle dont une ou plusieurs porte(nt) un substituant hydroxyalkyle.

**16.** Composition anti-rides selon la revendication 15, **caractérisée par le fait que** la molécule d'allongement de chaîne est choisie parmi l'acide diméthylolpropionique, l'acide $\alpha,\alpha$-bis(hydroxyméthyl)-butyrique, l'acide $\alpha,\alpha,\alpha$-tris(hydroxyméthyl)-acétique, l'acide $\alpha,\alpha$-bis(hydroxyméthyl)-valérique, l'acide $\alpha,\alpha$-bis(hydroxy)-propionique et l'acide 3,5-dihydroxybenzoïque.

**17.** Composition anti-rides selon l'une quelconque des revendications 12 à 16, **caractérisée par le fait que** le composé initiateur est choisi parmi le ditriméthylolpropane, le triméthylolpropane, un pentaérythritol éthoxylé, le pentaérythritol ou le glycérol, et que la molécule d'allongement de chaîne est l'acide diméthylolpropionique.

**18.** Composition anti-rides selon l'une quelconque des revendications 12 à 17, **caractérisée par le fait qu'**une partie des fonctions hydroxyle terminales du polymère dendritique de type polyester porte des substituants dérivés d'au moins un agent de terminaison de chaîne.

**19.** Composition anti-rides selon l'une quelconque des revendications précédentes, **caractérisée par le fait que** la concentration du polymère dendritique rapportée à la composition totale est comprise dans l'intervalle allant de 0,1 à 5 % en poids.

**20.** Composition anti-rides selon l'une quelconque des revendications précédentes, **caractérisée par le fait que** le système polymérique filmogène est présent à raison de 0,5 à 70 % en poids, de préférence de 0,5 à 30 % en poids, exprimé en matière active rapportée à la composition totale.

**21.** Composition anti-rides selon l'une quelconque des revendications précédentes, **caractérisée par le fait que** le rapport en poids du polymère dendritique au système polymérique filmogène est compris dans l'intervalle allant de 1/2 à 1/100, de préférence de 1/10 à 1/50.

**22.** Composition anti-rides selon l'une quelconque des revendications précédentes, **caractérisée par le fait qu'**elle contient en outre un ou plusieurs principes actifs cosmétiques choisis parmi les agents anti-radicaux libres, les agents hydratants, les vitamines, les protéines autres que les polymères tenseurs, les céramides, les $\alpha$-hydroxy-acides, les $\beta$-hydroxyacides et les rétinoïdes.

**23.** Composition anti-rides selon l'une quelconque des revendications précédentes, **caractérisée par le fait qu'**elle contient en outre au moins un adjuvant choisi parmi les solvants, les agents régulateurs de pH, les anti-oxydants, les agents conservateurs, les pigments et colorants, les charges, les émollients, les agents anti-mousse, les huiles ou cires végétales ou animales, les silicones, les parfums, les agents tensioactifs, les plastifiants, les polymères épaississants ou gélifiants et'les filtres solaires.

**24.** Composition anti-rides selon l'une quelconque des revendications précédentes, **caractérisée par le fait qu'**elle se présente sous forme d'une émulsion telle qu'une crème ou un lait, d'un gel, d'une lotion, d'une dispersion vésiculaire, d'un sérum, d'une pâte ou d'un bâtonnet solide.

**25.** Procédé de traitement non thérapeutique de la peau consistant à appliquer sur la peau du visage, du cou et/ou du décolleté une composition anti-rides selon l'une quelconque des revendications précédentes.

**26.** Utilisation cosmétique de la composition selon l'une quelconque des revendications 1 à 24 pour diminuer et/ou effacer les rides et/ou les ridules de la peau par un effet tenseur.

**27.** Utilisation d'une association

- d'une dispersion d'un système polymérique filmogène contenant au moins un polymère d'origine naturelle et/ou synthétique et capable de former un film perméable à la vapeur d'eau, présentant un module d'Young allant de $10^8$ à $9.10^{10}$ N/m$^2$ et produisant, après application à une concentration de 7 % dans l'eau puis séchage, une rétraction du *stratum corneum* isolé supérieure à 1 % à une température de 30 °C et pour une humidité relative de 40 %, et
- d'un polymère dendritique de type polyester à fonctions hydroxyle terminales,

pour la préparation d'une composition dermatologique destinée à diminuer et/ou effacer les rides et/ou ridules de la peau.

**Patentansprüche**

**1.** Kosmetische oder dermatologische Zusammensetzung gegen Falten, **dadurch gekennzeichnet, dass** sie in einem physiologisch akzeptablen Medium die Kombination

- einer Dispersion eines filmbildenden Polymersystems, das mindestens ein Polymer natürlichen und/oder synthetischen Ursprungs enthält und zur Bildung eines für Wasserdampf durchlässigen Films befähigt ist, der einen Young-Modul von $10^8$ bis $10^{10}$ N/m$^2$ aufweist und nach dem Auftragen in einer Konzentration von 7 % in Wasser und anschließendem Trocknen bei einer Temperatur von 30 °C und einer relativen Feuchte von 40 % eine Retraktion von isoliertem *stratum corneum* von mehr als 1 % hervorruft, und
- eines dendritischen Polymers vom Typ eines Polyesters mit endständigen Hydroxygruppen

enthält.

**2.** Zusammensetzung gegen Falten nach Anspruch 1, **dadurch gekennzeichnet, dass** es sich bei dem in dem filmbildenden Polymersystem enthaltenen Polymer um ein Polymer natürlichen Ursprungs handelt.

**3.** Zusammensetzung gegen Falten nach Anspruch 2, **dadurch gekennzeichnet, dass** das Polymer natürlichen Ursprungs unter Polymeren pflanzlichen Ursprungs, aus Hautanhangsgebilden stammenden Polymeren, Proteinen von Eiern und Latices natürlichen Ursprungs ausgewählt ist.

**4.** Zusammensetzung gegen Falten nach Anspruch 3, **dadurch gekennzeichnet, dass** das Polymer natürlichen Ursprungs vom Typ eines Latex unter Schellackharz, Sandarak, Dammarharzen, Elemiharzen, Kopalen, Cellulosederivaten und den Gemischen dieser Polymere ausgewählt ist.

**5.** Zusammensetzung gegen Falten nach Anspruch 3, **dadurch gekennzeichnet, dass** das aus Hautanhangsgebilden stammende Polymer unter Chitin, Chitosan, Hydroxypropylchitosan, dem succinylierten Derivat von Chitosan, Chitosanlactat, Chitosanglutamat, Carboxymethylchitosansuccinamid, Keratinhydrolysaten und Sulfokeratinen ausgewählt ist.

**6.** Zusammensetzung gegen Falten nach Anspruch 3, **dadurch gekennzeichnet, dass** das Polymer pflanzlichen Ursprungs unter proteinhaltigen Extrakten von Getreide, Hülsenfrüchten und Öl liefernden Pflanzen ausgewählt ist.

**7.** Zusammensetzung gegen Falten nach Anspruch 6, **dadurch gekennzeichnet, dass** der Extrakt unter proteinhaltigen Extrakten von Mais, Roggen, Weizen, Buchweizen, Sesam, Dinkel, Erbsen, Bohnen, Linsen, Soja und Lupinen ausgewählt ist.

**8.** Zusammensetzung gegen Falten nach Anspruch 1, **dadurch gekennzeichnet, dass** es sich bei dem in dem filmbildenden Polymersystem enthaltenen Polymer um ein synthetisches Polymer in Form eines Latex oder Pseudolatex handelt.

**9.** Zusammensetzung gegen Falten nach Anspruch 8, **dadurch gekennzeichnet, dass** das synthetische Polymer unter anionischen, kationischen, nichtionischen oder amphoteren Polyurethanen, Polyacryl-Polyurethanen, Polyurethan-Polyvinylpyrrolidonen, Polyurethan-Polyestern, Polyurethan-Polyethern, Polyharnstoffen, Acrylhomopolymeren oder Acrylcopolymeren, Polymeren von Sulfoisophthalsäure und den Gemischen dieser Verbindungen ausgewählt ist.

**10.** Zusammensetzung gegen Falten nach einem der vorhergehenden Ansprüche, **dadurch gekennzeichnet, dass** das in dem filmbildenden Polymersystem enthaltene Polymer synthetischen und/oder natürlichen Ursprungs freie Hydroxygruppen oder anionische Gruppen aufweist.

**11.** Zusammensetzung gegen Falten nach einem der vorhergehenden Ansprüche, **dadurch gekennzeichnet, dass** das Polymersystem ferner einen Weichmacher enthält.

**12.** Zusammensetzung gegen Falten nach einem der vorhergehenden Ansprüche, **dadurch gekennzeichnet, dass** es sich bei dem dendritischen Polymer mit endständigen Hydroxygruppen um ein stark verzweigtes Makromolekül vom Typ eines Polyesters handelt, das aus

- einer Zentraleinheit, die von einer Initiatorverbindung mit einer oder mehreren Hydroxygruppen (a) abgeleitet ist, und
- Einheiten zur Verlängerung der Kette, die von einem Molekül zur Verlängerung der Kette mit einer Carboxygruppe (b) und mindestens zwei Hydroxygruppen (c) abgeleitet sind,

besteht, wobei die einzelnen Hydroxygruppen (a) des zentralen Moleküls den Ausgangspunkt für eine Polykondensationsreaktion (Polyveresterung) darstellen, die mit der Umsetzung der Hydroxygruppen (a) des zentralen Moleküls mit den Carboxygruppen (b) der Moleküle zur Verlängerung der Kette beginnt und sich anschließend mit der Umsetzung der Carboxygruppen (b) mit den Hydroxygruppen (c) der Moleküle zur Verlängerung der Kette fortsetzt.

**13.** Kosmetische Zusammensetzung gegen Falten nach Anspruch 12, **dadurch gekennzeichnet, dass** die Initiatorverbindung mit einer oder mehreren Hydroxygruppen, die die Zentraleinheit bildet, ausgewählt ist unter

(a) einem monofunktionellen Alkohol,
(b) einem aliphatischen, cycloaliphatischen oder aromatischen Diol,
(c) einem Triol,

(d) einem Tetrol,

(e) einem Zuckeralkohol,

(f) Anhydroenneaheptit oder Dipentaerythrit,

(g) einem $\alpha$-Alkylglykosid und

(h) einem polyalkoxylierten Polymer mit einer Molmasse von höchstens 8 000, das durch Polyalkoxylierung eines der Alkohole (a) bis

(g) erhalten wird.

14. Kosmetische Zusammensetzung gegen Falten nach Anspruch 13, **dadurch gekennzeichnet, dass** die Initiatorverbindung unter Ditrimethylolpropan, Ditrimethylolethan, Dipentaerythrit, Pentaerythrit, einem alkoxylierten Pentaerythrit, Trimethylolethan, Trimethylolpropan, einem alkoxylierten Trimethylolpropan, Glycerin, Neopentylglykol, Dimethylolpropan oder 1,3-Dioxan-5,5-dimethanol ausgewählt ist.

15. Kosmetische Zusammensetzung gegen Falten nach einem der Ansprüche 12 bis 14, **dadurch gekennzeichnet, dass** das Molekül zur Verlängerung der Kette ausgewählt ist unter

- Monocarbonsäuren mit mindestens zwei Hydroxygruppen und
- Monocarbonsäuren mit mindestens zwei Hydroxygruppen, von denen eine oder mehrere einen Hydroxyalkylsubstituenten tragen.

16. Zusammensetzung gegen Falten nach Anspruch 15, **dadurch gekennzeichnet, dass** das Molekül zur Verlängerung der Kette unter Dimethylolpropionsäure, $\alpha,\alpha$-Bis(hydroxymethyl)-buttersäure, $\alpha,\alpha,\alpha$-Tris(hydroxymethyl)-essigsäure, $\alpha,\alpha$-Bis(hydroxymethyl)-valeriansäure, $\alpha,\alpha$-Bis(hydroxy)-propionsäure und 3,5-Dihydroxybenzoesäure ausgewählt ist.

17. Zusammensetzung gegen Falten nach einem der Ansprüche 12 bis 16, **dadurch gekennzeichnet, dass** die Initiatorverbindung unter Dimethylolpropan, Trimethylolpropan, einem ethoxylierten Pentaerythrit, Pentaerythrit oder Glycerin ausgewählt ist und dass das Molekül zur Verlängerung der Kette Dimethylolpropionsäure ist.

18. Zusammensetzung gegen Falten nach einem der Ansprüche 12 bis 17, **dadurch gekennzeichnet, dass** ein Teil der endständigen Hydroxygruppen des dendritischen Polymers vom Typ eines Polyesters Substituenten trägt, die von mindestens einem Mittel zum Kettenabbruch abgeleitet sind.

19. Zusammensetzung gegen Falten nach einem der vorhergehenden Ansprüche, **dadurch gekennzeichnet, dass** die Konzentration des dendritischen Polymers, bezogen auf die gesamte Zusammensetzung, im Bereich von 0,1 bis 5 Gew.-% liegt.

20. Zusammensetzung gegen Falten nach einem der vorhergehenden Ansprüche, **dadurch gekennzeichnet, dass** das filmbildende Polymersystem in einem als Wirkstoff ausgedrückten Mengenanteil von 0,5 bis 70 Gew.-% und vorzugsweise von 0,5 bis 30 Gew.-%, bezogen auf die gesamte Zusammensetzung, vorliegt.

21. Zusammensetzung gegen Falten nach einem der vorhergehenden Ansprüche, **dadurch gekennzeichnet, dass** das Gewichtsverhältnis des dendritischen Polymers zu dem filmbildenden Polymersystem im Bereich von 1/2 bis 1/100 und vorzugsweise von 1/10 bis 1/50 liegt.

22. Zusammensetzung gegen Falten nach einem der vorhergehenden Anspruche, **dadurch gekennzeichnet, dass** sie ferner einen oder mehrere kosmetische Wirkstoffe enthält, die unter Radikalfängern gegen freie Radikale, Hydratisierungsmitteln, Vitaminen, Proteinen, die von den straffenden Polymeren verschieden sind, Ceramiden, $\alpha$-Hydroxysäuren, $\beta$-Hydroxysäuren und Retinoiden ausgewählt sind.

23. Zusammensetzung gegen Falten nach einem der vorhergehenden Ansprüche, **dadurch gekennzeichnet, dass** sie ferner mindestens einen Zusatzstoff enthält, der unter Lösemitteln, Mitteln zum Regulieren des pH-Werts, Antioxidantien, Konservierungsstoffen, Pigmenten oder Farbstoffen, Füllstoffen, Emollentien, Mitteln gegen Schaumbildung, pflanzlichen oder tierischen Ölen oder Wachsen, Siliconen, Parfums, grenzflächenaktiven Stoffen, Weichmachern, verdickenden oder gelierenden Polymeren und Sonnenschutzfiltern ausgewählt ist.

24. Zusammensetzung gegen Falten nach einem der vorhergehenden Ansprüche, **dadurch gekennzeichnet, dass** sie in Form einer Emulsion, wie einer Creme oder einer Milch, eines Gels, einer Lotion, einer Vesikeldispersion, eines Serums, einer Paste oder eines festen Stifts vorliegt.

25. Verfahren zur nichttherapeutischen Behandlung der Haut, das darin besteht, eine Zusammensetzung gegen Falten nach einem der vorhergehenden Ansprüche auf die Haut des Gesichts, des Halses und/oder des Dekolletés aufzubringen.

26. Kosmetische Verwendung der Zusammensetzung nach einem der Ansprüche 1 bis 24, um Hautfalten und/oder Hautfältchen durch einen Straffungseffekt abzuschwächen und/oder zu beseitigen.

27. Verwendung einer Kombination

- einer Dispersion eines film bildenden Polymersystems, das mindestens ein Polymer natürlichen und/oder synthetischen Ursprungs enthält und zur Bildung eines für Wasserdampf durchlässigen Films befähigt ist, der einen Young-Modul von $10^8$ bis $9 \cdot 10^{10}$ N/m$^2$ aufweist und nach dem Auftragen in einer Konzentration von 7 % in Wasser und anschließendem Trocknen bei einer Temperatur von 30 °C und eine relativen Feuchte von 40 % eine Retraktion von isoliertem *stratum corneum* von mehr als 1 % hervorruft, und
- eines dendritischen Polymers vom Typ eines Polyesters mit endständigen Hydroxygruppen

zur Herstellung einer dermatologischen Zusammensetzung, die dazu bestimmt ist, Hautfalten und/oder Hautfältchen abzuschwächen und/oder zu beseitigen.

**Claims**

1. Antiwrinkle cosmetic or dermatological composition, **characterized in that** it comprises, in a physiologically acceptable medium, the combination

- of a dispersion of a film-forming polymeric system containing at least one polymer of natural and/or synthetic origin and capable of forming a film permeable to water vapour, having a Young's modulus ranging from $10^8$ to $10^{10}$ N/m$^2$ and producing, after application at a concentration of 7% in water and then drying, a retraction of the isolated *stratum corneum* greater than 1% at a temperature of 30°C and for a relative humidity of 40%, and
- of a dendritic polymer of the polyester type with terminal hydroxyl functions.

2. Antiwrinkle composition according to Claim 1, **characterized in that** the polymer contained in the said film-forming polymeric system is a polymer of natural origin.

3. Antiwrinkle composition according to Claim 2, **characterized in that** the polymer of natural origin is chosen from the polymers of plant origin, the polymers derived from superficial body growths, egg proteins and latexes of natural origin.

4. Antiwrinkle composition according to Claim 3, **characterized in that** the polymer of natural origin of the latex type is chosen from shellac resin, sandarac gum, dammars, elemis, copals, cellulose derivatives and mixtures of these polymers.

5. Antiwrinkle composition according to Claim 3, **characterized in that** the polymer derived from superficial body growths is chosen from chitin, chitosan, hydroxypropylchitosan, the succinylated derivative of chitosan, chitosan lactate, chitosan glutamate, carboxymethylchitosan succinamide, keratin hydrolysates and sulphonic keratins.

6. Antiwrinkle composition according to Claim 3, **characterized in that** the polymer of plant origin is chosen from protein extracts of cereals, legumes and oilseeds.

7. Antiwrinkle composition according to Claim 6, **characterized in that** the extract is chosen from the protein extracts of maize, rye, wheat, buckwheat, sesame, spelt, pea, broadbean, lentil, soyabean and lupin.

8. Antiwrinkle composition according to Claim 1, **characterized in that** the polymer contained in the said film-forming polymeric system is a synthetic polymer provided in the form of a latex or pseudolatex.

9. Antiwrinkle composition according to Claim 8, **characterized in that** the synthetic polymer is chosen from anionic, cationic, nonionic or amphoteric polyurethanes, polyurethane-polyacrylics, polyurethane-polyvinylpyrrolidones, polyurethane-polyesters, polyurethane-polyethers, polyureas, acrylic homo- or copolymers, polymers of sulphonated isophthalic acid and mixtures thereof.

10. Antiwrinkle composition according to any one of the preceding claims, **characterized in that** the polymer of synthetic and/or natural origin contained in the said film-forming polymeric system contains free hydroxyl groups or anionic groups.

11. Antiwrinkle composition according to any one of the preceding claims, **characterized in that** the polymeric system comprises, in addition, a plasticizing agent.

12. Antiwrinkle composition according to any one of the preceding claims, **characterized in that** the dendritic polymer with terminal hydroxyl functions is a highly branched macromolecule of the polyester type, consisting

- of a central unit derived from an initiator compound carrying one or more hydroxyl functions (a),
- chain-extension units derived from a chain-extension molecule carrying a carboxyl function (b) and at least two hydroxyl functions (c),

each of the hydroxyl functions (a) of the central molecule being the starting point of a polycondensation reaction (polyesterification) which starts with the reaction of the hydroxyl functions (a) of the central molecule with the carboxyl functions (b) of the chain-extension molecules, and then continues with the reaction of the carboxyl functions (b) with the hydroxyl functions (c) of the chain-extension molecules.

13. Antiwrinkle cosmetic composition according to Claim 12, **characterized in that** the- initiator compound carrying one or more hydroxyl functions forming the central unit is chosen from

(a) a monofunctional alcohol,
(b) an aliphatic, cycloaliphatic or aromatic diol,
(c) a triol,
(d) a tetrol,
(e) a sugar alcohol,
(f) anhydro-ennea-heptitol or dipentaerythritol,
(g) an $\alpha$-alkylglycoside,
(h) a polyalkoxylated polymer obtained by polyalkoxylation of one of the alcohols (a) to (g), having a molar mass at most equal to 8000.

14. Antiwrinkle cosmetic composition according to Claim 13, **characterized in that** the initiator compound is chosen from ditrimethylolpropane, ditrimethylolethane, dipentaerythritol, pentaerythritol, an alkoxylated pentaerythritol, trimethylolethane, trimethylolpropane, an alkoxylated trimethylolpropane, glycerol, neopentyl glycol, dimethylolpropane or 1,3-dioxane-5,5-dimethanol.

15. Antiwrinkle cosmetic composition according to any one of Claims 12 to 14, **characterized in that** the chain-extension molecule is chosen from

- monocarboxylic acids containing at least two hydroxyl functions, and
- monocarboxylic acids containing at least two hydroxyl functions of which one or more carry a hydroxyalkyl substituent.

16. Antiwrinkle composition according to Claim 15, **characterized in that** the chain-extension molecule is chosen from dimethylolpropionic acid, $\alpha,\alpha$-bis(hydroxymethyl)butyric acid, $\alpha,\alpha,\alpha$-tris(hydroxymethyl)acetic acid, $\alpha,\alpha$-bis(hydroxymethyl)valeric acid, $\alpha,\alpha$-bis(hydroxy)propionic acid and 3,5-dihydroxybenzoic acid.

17. Antiwrinkle composition according to any one of Claims 12 to 16, **characterized in that** the initiator compound is chosen from ditrimethylolpropane, trimethylolpropane, an ethoxylated pentaerythritol, pentaerythritol or glycerol, and **in that** the chain-extension molecule is dimethylolpropionic acid.

18. Antiwrinkle composition according to any one of Claims 12 to 17, **characterized in that** some of the terminal hydroxyl functions of the polyester-type dendritic polymer carry substituents derived from at least one chain-terminating agent.

19. Antiwrinkle composition according to any one of the preceding claims, **characterized in that** the concentration of the dendritic polymer relative to the total composition is within the interval ranging from 0.1 to 5% by weight.

20. Antiwrinkle composition according to any one of the preceding claims, **characterized in that** the film-forming polymeric system is present in an amount of 0.5 to 70% by weight, preferably 0.5 to 30% by weight, expressed as active material relative to the total composition.

21. Antiwrinkle composition according to any one of the preceding claims, **characterized in that** the weight ratio of the dendritic polymer to the film-forming polymeric system is within the interval ranging from 1/2 to 1/100, preferably from 1/10 to 1/50.

22. Antiwrinkle composition according to any one of the preceding claims, **characterized in that** it contains, in addition, one or more cosmetic active ingredients chosen from anti-free radical agents, moisturizing agents, vitamins, proteins other than the tightening polymers, ceramides, $\alpha$-hydroxy acids, $\beta$-hydroxy acids and retinoids.

23. Antiwrinkle composition according to any one of the preceding claims, **characterized in that** it contains, in addition, at least one adjuvant chosen from solvents, pH-regulating agents, antioxidants, preservatives, pigments and colourings, fillers, emollients, antifoams, vegetable or animal oils or waxes, silicones, perfumes, surfactants, plasticizers, thickening or gelling polymers and sunscreens.

24. Antiwrinkle composition according to any one of the preceding claims, **characterized in that** it is provided in the form of an emulsion such as a cream or a milk, a gel, a lotion, a vesicular dispersion, a serum, a paste or a solid stick.

25. Method of nontherapeutic treatment of the skin consisting in applying to the skin of the face, the neck and/or the cleavage an antiwrinkle composition according to any one of the preceding claims.

26. Cosmetic use of the composition according to any one of Claims 1 to 24 for reducing and/or removing the wrinkles and/or fine lines on the skin by a tightening effect.

27. Use of a combination

- of a dispersion of a film-forming polymeric system containing at least one polymer of natural and/or synthetic origin and capable of forming a film permeable to water vapour, having a Young's modulus ranging from $10^8$ to $9\times10^{10}$ N/m$^2$ and producing, after application at a concentration of 7% in water and then drying, a retraction of the isolated *stratum corneum* greater than 1% at a temperature of 30°C and for a relative humidity of 40%, and
- of a dendritic polymer of the polyester type with terminal hydroxyl functions,

for the preparation of a dermatological composition intended for reducing and/or removing the wrinkles and/or fine lines on the skin.